# EUROPÄISCHE PATENTSCHRIFT

(11) **EP 4 157 158 B1**
(45) Veröffentlichungstag und Bekanntmachung des Hinweises auf die Patenterteilung: **02.12.2026**
(21) Anmeldenummer: 21725725.2
(22) Anmeldetag: 06.05.2021
(51) Int. Cl.: A61F 2/50, A61F 5/01

(54) **VERFAHREN ZUM FESTLEGEN EINER ORTHOPÄDIETECHNISCHEN EINRICHTUNG UND ORTHOPÄDIETECHNISCHE EINRICHTUNG**
METHOD FOR SECURING AN ORTHOPAEDIC DEVICE AND ORTHOPAEDIC DEVICE
MÉTHODE DE FIXATION D'UN DISPOSITIF ORTHOPÉDIQUE ET DISPOSITIF ORTHOPÉDIQUE

(30) Priorität: 27.05.2020 DE 102020114185
(43) Veröffentlichungstag der Anmeldung: 05.04.2023
(73) Patentinhaber: Ottobock SE & Co. KGaA, 37115 Duderstadt (DE)
(72) Erfinder: POHL, Kathrin, 37083 Goettingen (DE)
(74) Vertreter: Gramm, Lins & Partner Patent- und Rechtsanwälte PartGmbB
(86) Internationale Anmeldenummer: PCT/EP2021/062078
(87) Internationale Veröffentlichungsnummer: WO 2021/239436

(56) Entgegenhaltungen:
- WO-A1-2017/191120
- US-A- 5 743 866
- US-A1- 2007 239 093
- US-A1- 2019 083 285

## Beschreibung

Die Erfindung betrifft ein Verfahren zum Festlegen einer orthopädietechnischen Einrichtung an einem Körperteil eines Trägers. Orthopädietechnische Einrichtungen im Sinne der vorliegenden Erfindung sind insbesondere Prothesen und Orthesen. Aber auch Vorrichtungen, die den Träger beispielsweise bei schwerer körperlicher Arbeit, beispielsweise bei Arbeiten über Kopf, unterstützen sollen, werden als orthopädietechnische Einrichtungen angesehen.

Derartige Einrichtungen werden an einem oder mehreren Körperteilen festgelegt. Um die nötige Stabilität, Unterstützung, Schutzwirkung oder therapeutische Wirkung zu erreichen, ist es oftmals notwendig, eine gewisse Spannung auf das jeweilige Körperteil, an dem die Einrichtung angeordnet ist, aufzubringen. Dies geschieht beispielsweise über Gurte, die zumindest teilweise um das jeweilige Körperteil herumgelegt werden. Insbesondere Gelenkorthesen werden auf diese Weise am jeweiligen Gelenk festgelegt. Über die Gurte wird es möglich, die Spannung einzustellen und so eine einzige Orthese für unterschiedliche Personen und Träger der Orthese verwenden zu können, ohne individuelle Orthesen herstellen zu müssen. Gleiches gilt für Prothesen, die beispielsweise einen Prothesenschaft aufweisen, dessen Weite, also insbesondere die Öffnung des Prothesenschaftes, in die der Amputationsstumpf eingeführt wird, mittels eines Gurtes einstellbar ist.

Eine orthopädietechnische Einrichtung verfügt dabei über einen Grundkörper, der an dem jeweiligen Körperteil positioniert wird. Die orthopädietechnische Einrichtung verfügt zudem über wenigstens einen Gurt, der zumindest teilweise um das jeweilige Körperteil herumgelegt und befestigt wird. Dazu befindet sich am Grundkörper zumeist eine Art Schlaufe oder Öse, die aus einem Kunststoff oder einem Metall hergestellt ist. Der Gurt verfügt über ein freies Ende, dass durch diese Schlaufe oder Öse hindurch geführt wird, bevor er beispielsweise auf sich selbst festgelegt wird. Dazu verfügt der Gurt an seinem Ende beispielsweise über ein erstes Formschlusselement, beispielsweise ein Klettverschlusselement, das mit einem zweiten dazu korrespondierend ausgebildeten Formschlusselement zusammenwirken kann, das beispielsweise ebenfalls an dem Gurt angeordnet ist. Die beiden Formschlusselemente werden dann so aneinander angeordnet, dass eine formschlüssige Verbindung entsteht. Je nachdem, wie weit das freie Ende des Gurtes durch die Schlaufe oder Öse hindurch geführt wird, kann die Weite des Gurtes frei eingestellt werden, solange die beiden Formschlusselemente miteinander in Kontakt kommen. Eine solche Ausgestaltung ist aus der US 2007/0239093 A1 bekannt. Eine ähnliche Ausgestaltung wird in der WO 2017/191120 A 1 und der US-5743866-A beschrieben.

Die US 2019/0083285 A1 beschreibt eine Ausgestaltung, bei der am Ende des Gurtes eine Schlaufe positioniert ist, die um ein Formschlusselement, das beispielsweise als Stift ausgelegt sein kann, herumgelegt wird.

Nachteilig ist jedoch, dass das Einfädeln des freien Endes des Gurtes in die Schlaufe oder Öse eine relativ hohe Fingerfertigkeit erfordert, die insbesondere bei Trägern der orthopädietechnischen Einrichtung, die motorisch eingeschränkt sind, nicht immer vorhanden ist. Zudem hängt die Position der Öse oder Schlaufe stark davon ab, an welchem Körperteil die orthopädietechnische Einrichtung angeordnet werden soll. So ist es zusätzlich zu der körperlichen Einschränkung der Träger oftmals schwierig, die Position zu erreichen, an der sich die Schlaufe oder Öse befindet. Soll nun das freie Ende des Gurtes durch die Öse oder Schlaufe hindurchgefädelt werden, muss das freie Ende zunächst hindurchgesteckt, dann mit der zweiten Hand ergriffen und weiterhin durchgezogen werden. Mit einer einzigen Hand ist dies nur sehr schwer oder gar nicht möglich.

Der Erfindung liegt daher die Aufgabe zugrunde, ein Verfahren zum Festlegen einer orthopädietechnischen Einrichtung an einem Körperteil eines Trägers vorzuschlagen, das einfach durchzuführen ist und dennoch eine individuelle Einstellbarkeit und eine ausreichende Sicherheit der Verbindung ermöglicht. Der Erfindung liegt zudem die Aufgabe zugrunde, eine entsprechende orthopädietechnische Einrichtung vorzuschlagen.

Die Erfindung löst die gestellte Aufgabe durch ein Verfahren zum Festlegen einer orthopädietechnischen Einrichtung an einem Körperteil eines Trägers, wobei die Einrichtung einen Grundkörper, von dem wenigstens ein Umlenkelement mit einer Anlagefläche in einer Richtung vom Körperteil des Trägers wegweisend hervorsteht, wenigstens einen Gurt und wenigstens ein Befestigungselement aufweist, wobei das Verfahren folgende Schritte aufweist:
a) Positionieren der orthopädietechnischen Einrichtung an dem Körperteil,
b) Anlegen des Gurtes an die Anlagefläche des Umlenkelementes, ohne dass dazu ein Ende des Gurtes durch eine Schlaufe oder Öse gefädelt werden muss, und
c) Befestigen des Gurtes an dem Befestigungselement, so dass sich der Gurt entlang zweier Teilstrecken erstreckt,

wobei die erste Teilstrecke von einem festen Ende des Gurtes, mit dem er an einem Bauteil oder Element der orthopädietechnischen Einrichtung festgelegt ist, bis zu dem Umlenkelement und
wobei die zweite Teilstrecke von dem Umlenkelement bis zu dem Befestigungselement verläuft.

Durch das erfindungsgemäße Verfahren ist es möglich, die orthopädietechnische Einrichtung an dem Körperteil des Trägers festzulegen, ohne, dass dazu ein Ende des Gurtes durch eine Schlaufe oder Öse gefädelt werden muss. Dadurch wird das Verfahren einfacher, schneller und auch für Personen mit motorischen Einschränkungen leicht durchführbar.

Das wenigstens eine Umlenkelement steht von dem Grundkörper hervor. Dies bedeutet insbesondere, dass es in eine Richtung vom Körperteil des Trägers wegweisend, hervorsteht. Das Umlenkelement ist beispielsweise ein zylinderförmiges Element, das eine umlaufende Mantelfläche aufweist. Ein Teil dieser Mantelfläche oder die gesamte Mantelfläche bildet dabei die Anlagefläche. Es ist aber nicht notwendig, dass das Umlenkelement eine Symmetrieachse oder eine Rotationssymmetrie aufweist, kann jedoch von Vorteil sein. Das Umlenkelement verfügt vorzugsweise über einen Rand, der die Anlagefläche auf der vom Grundkörper abgewandten Seite begrenzt. Der Abstand dieses Randes vom Grundkörper kann dabei variieren.

Zum Durchführen des Verfahrens wird zunächst die orthopädietechnische Einrichtung an dem Körperteil angeordnet. Anschließend greift der Träger vorzugsweise ein freies Ende des Gurtes und führt damit eine Bewegung aus, durch die der Gurt an der Anlagefläche des Umlenkelementes angelegt wird. Dazu ist es nicht notwendig, den Gurt durch ein geschlossenes Element, beispielsweise eine Schlaufe oder eine Öse, zu führen. Der Gurt muss auch nicht von einer Hand in die andere übergeben werden. Die durchzuführende Bewegung ist vorzugsweise eine kreisförmige Bewegung, durch die ein Teil des Gurtes über einen Teil des Umlenkelementes an der dort befindlichen Anlagefläche angelegt wird. Dies ist einfach, schnell und sicher möglich und bietet dem Träger zudem die Möglichkeit, durch die von ihm aufzubringende Zugkraft die Spannung, die auf den Gurt wirken soll, frei einzustellen. Anschließend wird der Gurt an dem Befestigungselement befestigt.

Durch die erfindungsgemäße Ausgestaltung der orthopädietechnischen Einrichtung und des Verfahrens zum Festlegen dieser Einrichtung ist es zudem möglich, auf besonders einfache Weise, die eingestellte Spannung, die durch die aufgebrachte Zugkraft erzeugt wird, einzustellen und gegebenenfalls zu verändern. Dies kann beispielsweise von Vorteil sein, wenn sich ein Umfang des Körperteils, an dem die orthopädietechnische Einrichtung festgelegt ist, beispielsweise im Laufe eines Tages ändert. Auch wenn der Träger nach einiger Zeit merkt, dass die ursprünglich eingestellte Spannung zu groß oder zu klein ist, kann korrigiert werden. Vorzugsweise wird dazu zunächst der Gurt von dem Befestigungselement gelöst. Der Träger kann dann den Gurt von der Anlagefläche des Umlenkelementes entfernen und mit einer neuen Spannung, die größer oder kleiner als die ursprüngliche Spannung sein kann, erneut an der Anlagefläche positionieren. Anschließend wird der Gurt erneut an dem Befestigungselement befestigt. Alternativ oder zusätzlich dazu ist es auch möglich, die Spannung zu ändern, ohne den Gurt von der Anlagefläche des Umlenkelementes zu entfernen. Soll die Spannung beispielsweise erhöht werden, kann der Träger nach dem Lösen des Gurtes von dem Befestigungselement eine Zugkraft aufbringen, die dafür sorgt, dass der Gurt an der Anlagefläche des Umlenkelementes entlanggleitet. Dadurch wird die Spannung des Gurtes erhöht. In diesem Zustand kann der Gurt wieder an dem Befestigungselement befestigt werden. Alternativ dazu kann der Träger auch nach dem Lösen des Gurtes von dem Befestigungselement die Zugkraft reduzieren und damit dafür sorgen, dass der Gurt in die entgegengesetzte Richtung an der Anlagefläche des Umlenkelementes entlanggleitet. Sobald die Spannung auf den gewünschten Wert eingestellt ist, kann der Gurt wieder an dem Befestigungselement befestigt werden.

Der Gurt ist vorzugsweise aus einem elastischen Material hergestellt oder verfügt zumindest über einen, bevorzugt jedoch über mehrere Abschnitte, die aus einem elastischen Material hergestellt sind. Alternativ dazu ist der Gurt aus einem elastischen Material hergestellt.

In einer bevorzugten Ausgestaltung verfügt die Anlagefläche über eine Nut oder eine Hinterschneidung, in der der Gurt angelegt wird. Dadurch wird gewährleistet, dass der Gurt, wenn er einmal an der Anlagefläche angelegt ist, nicht von dem Umlenkelement abrutschen oder auf sonstige Weise versehentlich gelöst werden kann. Der Gurt liegt in der Vertiefung des Anlageelementes und kann erst nach dem Lösen des Gurtes von dem Befestigungselement entfernt werden. Dadurch wird die Sicherheit der Verbindung und damit der Tragekomfort erhöht.

Die Anlagefläche des Umlenkelementes erstreckt sich vorzugsweise, jedoch nicht notwendigerweise, über den gesamten Umfang des Umlenkelementes. Es ist ausreichend, eine Anlagefläche in dem Bereich des Umlenkelementes anzuordnen, der tatsächlich mit dem Gurt in Kontakt kommen soll. Die Größe der Anlagefläche, also insbesondere die Größe des Bereiches des Umfangs des Umlenkelementes, über den sich die Anlagefläche erstreckt, hängt dabei stark von der Position des Befestigungselementes ab. Zudem hat die Position, an der der Gurt mit seinem dem freien Ende gegenüberliegenden Ende an einem Element der orthopädietechnischen Einrichtung, vorzugsweise dem Grundkörper, angeordnet ist, einen Einfluss auf die Größe und Position der Anlagefläche am Umlenkelement.

**In** einer bevorzugten Ausgestaltung der Erfindung wird der Gurt derart an die Anlagefläche angelegt, dass der Gurt das Umlenkelement über wenigstens 25 %, bevorzugt wenigstens 40 %, besonders bevorzugt wenigstens 50 % seines Umfangs umgibt. Je größer der Teil der Anlagefläche ist, der tatsächlich mit dem Gurt in Kontakt kommt, desto größer ist auch die Fläche, an der die von dem Gurt auf das Umlenkelement übertragene Kraft übertragen wird. Damit reduziert sich der Druck, sodass die orthopädietechnische Einrichtung und insbesondere das Umlenkelement größeren Kräften standhalten können. Dadurch werden der konstruktive Aufwand und damit auch die Herstellungskosten reduziert.

Nachdem die orthopädietechnische Einrichtung an dem Körperteil des Trägers festgelegt wurde, erstreckt sich der Gurt entlang zweier Teilstrecken. Die erste Teilstrecke verläuft von dem festen Ende des Gurtes, mit dem er an einem Bauteil oder Element der orthopädietechnischen Einrichtung, insbesondere dem Grundkörper, festgelegt ist, bis zum Umlenkelement. Die zweite Teilstrecke verläuft vom Umlenkelement bis zum Befestigungselement, an dem der Gurt befestigt ist. Der Winkel zwischen diesen beiden Teilstrecken ist entscheidend dafür, wie viel Prozent des Umfangs des Umlenkelementes die Anlagefläche ausmacht.

**In** einer bevorzugten Ausgestaltung ist das Befestigungselement an dem Gurt angeordnet. **In** dieser Ausgestaltung wird der Gurt auf sich selbst festgelegt, wenn er an dem Befestigungselement befestigt wird. Der Winkel zwischen den beiden Teilstrecken beträgt in diesem Fall 180°. Die Anlagefläche erstreckt sich über etwa 50 % des Umfangs des Umlenkelementes. Alternativ oder zusätzlich dazu ist ein Befestigungselement t nicht am Gurt angeordnet. Es kann beispielsweise an einem anderen Element der orthopädietechnischen Einrichtung, beispielsweise am Grundkörper, angeordnet sein. **In** diesem Fall wird beim Befestigen des Gurtes der Gurt nicht auf sich selbst festgelegt, sodass der Winkel zwischen den beiden Teilstrecken größer oder kleiner als 180° sein kann. Ist der Winkel größer als 180° kreuzen sich die beiden Teilbereiche des Gurtes, die sich entlang der beiden Teilstrecken erstrecken. Ist der Winkel kleiner als 180° ist dies nicht der Fall.

Bei einer erfindungsgemäßen orthopädietechnischen Einrichtung wird durch den wenigstens einen Gurt in der Regel ein Zwischenraum überbrückt. So können beispielsweise zwei oder mehr Schalenelemente durch den wenigstens einen Gurt auf einen Umfang eingestellt werden. Der Zwischenraum kann jedoch auch zwischen den beiden Enden eines einzigen Grundkörpers, der beispielsweise in Form einer Spange ausgebildet sein kann, vorliegen. **In** einer bevorzugten Ausgestaltung ist das feste Ende des wenigstens einen Gurtes, das nicht an dem Befestigungselement befestigt wird, auf der einen Seite des Zwischenraums angeordnet, während sich das Umlenkelement auf der gegenüberliegenden Seite des Zwischenraums befindet. Insbesondere für den Fall, dass das Befestigungselement am Gurt angeordnet sind und der Gurt somit auf sich selbst zurückgelegt wird, wenn er am Befestigungselement befestigt wird, wird durch diese Anordnung nur in einem sehr kleinen Bereich, nämlich zwischen dem Befestigungselement einerseits und dem Umlenkelement andererseits eine Kraft aufgebracht. Oftmals erstreckt sich jedoch der Zwischenraum, der zu überbrücken ist, über eine größere Strecke, beispielsweise entlang eines Beines. Soll an mehreren Stellen oder über eine größere Strecke eine Kraft aufgebracht werden, kann dies erreicht werden, indem mehrere, voneinander beanstandete Gurte verwendet werden, die zum Festlegen der orthopädietechnischen Einrichtung jeweils an die Anlagefläche eines Umlenkelementes angelegt werden, bevor sie an jeweils einem Befestigungselement befestigt werden. Alternativ oder zusätzlich dazu kann jedoch auch ein Gurt um mehrere Umlenkelemente herumgelegt werden, die sich auf der gleichen oder auf verschiedenen Seiten des zu überbrückenden Zwischenraums befinden können.

So kann beispielsweise der Gurt, der mit seinem festen Ende auf einer Seite des zu überbrückenden Zwischenraums angeordnet ist, über den Zwischenraum geführt und an die Anlagefläche eines ersten Umlenkelementes angelegt werden. Anschließend verläuft der Gurt zu einem zweiten Umlenkelement, das sich auf der gleichen Seite des Zwischenraums befindet, wie das erste Umlenkelement. Auch hier wird der Gurt an eine Anlagefläche des zweiten Umlenkelementes angelegt, bevor er an dem Befestigungselement, das sich wieder auf der gegenüberliegenden Seite des Zwischenraums befindet, festgelegt wird. In dieser Ausgestaltung befinden sich das Befestigungselement und das feste Ende des Gurtes auf einer Seite des Zwischenraums und die beiden Umlenkelemente auf der anderen Seite des Zwischenraums.

In einer anderen Ausgestaltung ist der Gurt mit seinem festen Ende auf einer Seite des zu überbrückenden Zwischenraums angeordnet und wird über diesen Zwischenraum hinweg zum ersten Umlenkelement geführt. Dort wird er an die Anlagefläche des ersten Umlenkelementes angelegt und von dort zu einem zweiten Umlenkelement geführt, dass sich jedoch auf der anderen Seite des Zwischenraums befindet. Nachdem der Gurt auch an die Anlagefläche des zweiten Umlenkelementes angelegt wurde, wird er in diesem Ausführungsbeispiel wieder über den Zwischenraum geführt, und somit auf der gegenüberliegenden Seite mit dem sich dort befindenden Befestigungselement verbunden. In diesem Fall befinden sich folglich das Befestigungselement und das erste Umlenkelement auf einer Seite des Zwischenraums und das feste Ende des Gurtes und das zweite Umlenkelement auf der anderen Seite des Zwischenraums. Es ergibt sich ein Zickzack-Verlauf des Gurtes über den Zwischenraum. Selbstverständlich sind auch Kombinationen dieser Ausgestaltungen insbesondere mit mehr als zwei Umlenkelementen, beispielsweise drei, vier oder fünf Umlenkelementen, möglich.

Es können auch mehrere Umlenkelemente auf einer oder beiden Seiten des Zwischenraumes vorgesehen sein, von denen jeweils nur eines genutzt wird. Dies kann insbesondere hilfreich sein um eine Einstellbarkeit der aufgebrachten Spannung über einen größeren Bereich zu ermöglichen. Der Nutzer wählt dann das jeweils passende Umlenkelement aus.

Das erste Formschlusselement ist an dem zweiten Formschlusselement angeordnet, sodass eine formschlüssige Verbindung entsteht. Das erste Formschlusselement befindet sich vorzugsweise an einem freien Ende des Gurtes. Alternativ oder zusätzlich kann das Befestigungselement auch für eine reibschlüssige oder kraftschlüssige Verbindung ausgebildet sein. Das Befestigungselement verfügt beispielsweise über einen Schlitz, in dem der Gurt, der bevorzugt eine aufgeraute Oberfläche besitzt oder eine Beschichtung aufweist, eingefügt und somit eingeklemmt wird. Möglich ist auch, dass das Befestigungselement etwa in Form eines Knopfes oder eines pilzförmigen Objektes ausgebildet ist, das einen Abschnitt mit geringerem Durchmesser und einen breiteren Kopf aufweist. Der Gurt kann dann vorzugsweise mehrfach um den Abschnitt mit geringerem Durchmesser herumgelegt und auf diese Weise befestigt werden.

Die Erfindung löst die gestellte Aufgabe zudem durch eine orthopädietechnische Einrichtung zum Durchführen des Verfahrens der hier beschriebenen Art. Die Einrichtung verfügt über einen Grundkörper, von dem wenigstens ein Umlenkelement mit einer Anlagefläche hervorsteht, mindestens einen Gurt und wenigstens ein Befestigungselement, wobei der Gurt angeordnet und eingerichtet ist, an die Anlagefläche des Umlenkelementes angelegt und am Befestigungselement befestigt zu werden, ohne dass dazu ein Ende des Gurtes durch eine Schlaufe oder Öse gefädelt werden muss. Vorzugsweise verfügt der Gurt über ein erstes Formschlusselement und das Befestigungselement ist als ein dazu korrespondierend ausgebildetes zweites Formschlusselement ausgebildet, sodass dann das erste Formschlusselement mit dem zweiten Formschlusselement eine formschlüssige Verbindung bilden kann.

Vorzugsweise verfügt der Gurt über eine Breite, die in wenigstens einem Bereich, der mit der Anlagefläche in Kontakt kommen kann, reduziert ist. Insbesondere verfügt der Gurt in diesem Bereich über einen kreisförmigen oder nahezu kreisförmigen Querschnitt. Bevorzugt verfügt der Querschnitt in zwei senkrecht aufeinander stehenden Richtungen über ungefähr die gleiche Ausdehnung. Dieser Teil kommt mit der Anlagefläche des Umlenkelementes in Kontakt und wird um das Umlenkelement herumgelegt, sodass der Gurt sich in diesem Bereich nicht geradlinig erstreckt sondern in einer Kurve geführt wird. Dies ist mit einem Querschnitt, bei dem die Dicke etwa der Breite entspricht, besonders einfach möglich. Vorzugsweise verfügt der Gurt über mehrere derartige Bereiche, die bevorzugt durch wenigstens einen Bereich voneinander getrennt sind, in dem die Breite nicht reduziert ist. Der Benutzer kann dann durch Auswahl des zu verwendenden Bereiches mit reduzierter Breite grob die Spannung einstellen.

Verfügt der Gurt über eine konstante oder nahezu konstante Breite oder ist die Breite auch in dem Bereich, der mit der Anlagefläche in Kontakt kommen kann, deutlich größer als die Dicke des Gurtes an dieser Stelle, ist es von Vorteil, den Gurt beim Anlegen des Gurtes an die Anlagefläche des Umlenkelementes so zu drehen, dass die Seite des Gurtes, die in anderen Bereichen des Gurtes dem Körperteil des Trägers zugewandt ist, in dem Bereich, der mit der Anlagefläche in Kontakt kommen kann, der Anlagefläche zugewandt ist. Hierzu kann eine Drehung um etwa 90° von Vorteil sein.

Vorzugsweise ist die Breite in dem Bereich, der mit der Anlagefläche in Kontakt kommen kann, einseitig reduziert. Der Gurt verfügt über zwei Ränder, die den Gurt seitlich begrenzen. Der Abstand zwischen diesen beiden Rändern ist die Breite des Gurtes. Ist die Breite über den Verlauf des Gurtes konstant, verlaufen die Ränder parallel zueinander. Soll nun in einem Bereich die Breite des Gurtes verringert werden, kann dies auf unterschiedliche Weise geschehen. Eine Möglichkeit ist die symmetrische Reduzierung der Breite. Dies bedeutet, dass die Ränder jeweils aufeinander zu verlaufen, bis die gewünschte reduzierte Breite erreicht ist. In dem Bereich, der die reduzierte, dennoch jedoch konstante Breite aufweisen soll, verlaufen die Ränder nun im kleineren Abstand parallel zueinander, bevor sie sich wieder im weiteren Verlauf des Gurtes voneinander entfernen um eine größere, bevorzugt die ursprüngliche Breite zu erreichen. Alternativ zur symmetrischen Reduzierung der Breite gibt es auch die asymmetrische Reduzierung der Breite. Dies bedeutet, die beiden Ränder verlaufen nicht im gleichen Maße aufeinander zu. Dennoch verlaufen beide Ränder aufeinander zu, wenn auch unterschiedlich weit. Sobald der gewünschte Abstand, also die reduzierte Breite, erreicht ist, verlaufen auch in dieser Ausgestaltung die beiden Ränder parallel zueinander. Im weiteren Verlauf des Gurtes ist es von Vorteil, wenn die Ränder sich wieder voneinander entfernen und die Breite des Gurtes wieder zunimmt. Auch hierbei ist es von Vorteil, die ursprüngliche Breite wieder zu erreichen. In einer Draufsicht auf den Gurt verfügt dieser folglich über einen schmalen Abschnitt, der sich bei der symmetrischen Reduzierung genau in der Mitte des breiteren Abschnitts befindet, während er sich bei der asymmetrischen Reduzierung nicht in der Mitte befindet.

Eine weitere Möglichkeit ist die einseitige Reduzierung. Dabei verläuft nur einer der beiden Ränder auf den anderen zu, während der zweite Rand sich weiterhin geradlinig erstreckt. Der schmalere Abschnitt befindet sich somit am Rand des ursprünglichen breiteren Abschnittes. Diese Ausgestaltung ist bevorzugt, wobei sich besonders bevorzugt der schmalere Abschnitt des Gurtes an der dem Umlenkelement abgewandten Seite des Gurtes befindet. Wird also der Gurt so um das Umlenkelement herumgelegt, dass er von seinem festen Ende ausgehend in Richtung auf das erste Formschlusselement eine Rechtskurve bildet, befindet sich der schmalere Abschnitt am linken Rand des Gurtes. Wird der Gurt hingegen so um ein Umlenkelement herumgelegt, dass er von seinem festen Ende ausgehend in Richtung auf das erste Formschlusselement eine Linkskurve bildet, befindet sich der schmalere Abschnitt am rechten Rand des Gurtes. Verfügt die orthopädietechnische Einrichtung über mehrere Umlenkelemente, ist es von Vorteil, wenn auch der Gurt mehrere Bereiche mit einer reduzierten Breite aufweist. Besonders bevorzugt wird in jedem dieser Bereiche die einseitige Reduzierung der Breite verwendet, wobei sich der schmalere Abschnitt je nach Gurtführung am rechten oder linken Rand des Gurtes befindet, je nachdem, ob der Gurt eine Linkskurve oder eine Rechtskurve um das Umlenkelement herum bildet.

Vorzugsweise ist der Bereich mit reduzierter Breite länger als ein Kontaktbereich des Gurtes, mit dem der Gurt mit der Anlagefläche in Kontakt kommt. Vorzugsweise ist der Bereich mit reduzierter Breite wenigstens 1 cm, besonders bevorzugt wenigstens 2 cm länger. Dadurch lässt sich die Spannung sehr fein und nahezu stufenlos, besonders bevorzugt stufenlos, einstellen.

Vorzugsweise verfügt der Gurt über mehrere Bereiche mit reduzierter Breite. Der Nutzer kann dann auswählen, welchen dieser Bereiche er an das Umlenkelement anlegt und dadurch die Spannung über einen größeren Bereich variieren. Zunächst wählt er grob den Bereich aus, in dem die aufgebrachte Spannung liegen soll, indem er den Abschnitt mit reduzierte Breite auswählt, der mit dem Umlenkelement in Kontakt kommt. Anschließend kann er eine Feineinstellung wie oben beschrieben vornehmen.

**In** einer bevorzugten Ausgestaltung wird dieser Bereich durch ein Kunststoffelement, insbesondere ein Spritzgussteil gebildet. Es verfügt über die nötige Stabilität und Zugfestigkeit, und ist bevorzugt an andere Abschnitte des Gurtes angeklebt, angenäht oder eingeschweißt. Die anderen Abschnitte des Gurtes sind vorzugsweise aus einem Textil hergestellt und werden mit dem Kunststoffelement verbunden.

Vorzugsweise verfügt der Gurt über einen textilen Abschnitt, an dem das Kunststoffelement angeordnet ist. Besonders bevorzugt bildet das Kunststoffelement ein freies Ende des Gurtes. Dies bedeutet insbesondere, dass sich nur auf einer Seite des Kunststoffelementes ein textiler Abschnitt befindet. Verfügt der Gurt über ein Formschlusselement, ist dieses bevorzugt am Kunststoffelement angeordnet.

Vorzugsweise verfügt die orthopädietechnische Einrichtung über wenigstens zwei, bevorzugt mindestens drei oder vier Umlenkelemente, die jeweils eine Anlagefläche aufweisen und über den Grundkörper hervorstehen. Damit lassen sich die oben genannten Ausführungsformen realisieren und der Kraftverlauf, der durch den Gurt hervorgerufen werden soll, nahezu unbegrenzt einstellen.

In einer besonders bevorzugten Ausgestaltung ist das wenigstens eine Umlenkelement in Form eines Hakens ausgebildet.

Mithilfe der beiliegenden Zeichnungen werden nachfolgend einige Ausführungsbeispiele der vorliegenden Erfindung näher erläutert. Es zeigt
- Figur 1 bis 3 -: einen Teil einer orthopädietechnischen Einrichtung gemäß einem ersten Ausführungsbeispiel der vorliegenden Erfindung und
- Figur 4 -: einen Teil einer orthopädietechnischen Einrichtung gemäß einem weiteren Ausführungsbeispiel der vorliegenden Erfindung.

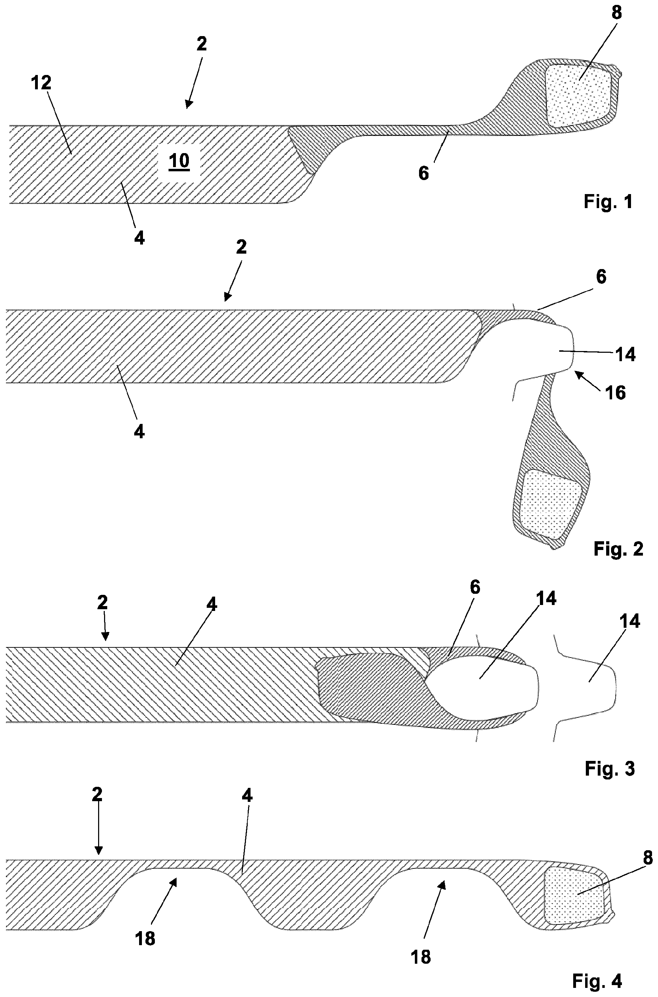

Figur 1 zeigt einen Gurt 2 für eine orthopädietechnische Einrichtung gemäß einem Ausführungsbeispiel der vorliegenden Erfindung. Er verfügt über einen textilen Anteil 4 und über ein Kunststoffelement 6, das an einem Ende des textilen Anteils 4 angeordnet ist. Am einen in Figur 1 rechten Ende des Kunststoffelementes 6, das gleichzeitig das rechte Ende des Gurtes 2 bildet, ist ein erstes Formschlusselement 8 angeordnet. Der textile Anteil 4 bildet eine zweites Formschlusselement 10 und damit das Befestigungselement 12, an dem der Gurt 2 befestigt wird.

Figur 2 zeigt den Gurt 2 mit dem textilen Abschnitt 4 und dem Kunststoffelement 6. Das Kunststoffelement 6 ist bereits teilweise um ein Umlenkelement 14 herumgelegt worden. Das Umlenkelement 14 verfügt über eine Hinterschneidung 16, in die das Kunststoffelement 6 mit dem Bereich reduzierter Breite eingelegt wird.

Figur 3 zeigt die Situation, in der das Ende des Kunststoffelementes 6, an dem sich das erste Formschlusselement 8 befindet, auf dem textilen Anteil 4 des Gurtes 2 befestigt ist. Dabei umgibt das Kunststoffelement 6 und damit der Gurt 2 zumindest teilweise das Umlenkelement 14. Durch die Hinterschneidung 16, in die der Gurt 2 mit dem Kunststoffelement 6 eingelegt ist, wird verhindert, dass der Gurt 2 vom Umlenkelement 14 abrutschen kann und die Verbindung so gelöst würde. In Figur 3 ist zudem ein zweites Umlenkelement 14 dargestellt, um das das Kunststoffelement 6 des Gurtes 2 ebenfalls hätte herumgelegt werden können. Dadurch wäre die orthopädietechnische Einrichtung unter stärkerer Spannung an einem Körperteil des Trägers der orthopädietechnischen Einrichtung, das nicht dargestellt ist, festgelegt worden.

Figur 4 zeigt einen Gurt 2 ohne Kunststoffelement 6. Der textile Anteil 4 erstreckt sich über die gesamte Länge. Am rechten Ende ist in Figur 4 das erste Formschlusselement 8 angeordnet. Anders als der Gurt 2 in den Figuren 1 bis 3 verfügt der Gurt 2 in Figur 4 über zwei Bereiche 18, in denen die Breite des Gurtes 2 reduziert ist. Es handelt sich um eine einseitige Reduzierung, da die Reduzierung nur an der in Figur 4 unteren Seite des Gurtes erfolgt ist. Der Gurt 2 kann in unterschiedlichen Positionen um ein Umlenkelement 14 herumgelegt werden. Jeder der beiden Bereiche 18 kann mit dem Umlenkelement 14 in Kontakt kommen, wodurch die Spannung eingestellt werden kann, unter der die orthopädietechnische Einrichtung, deren Teil der Gurt 2 ist, an einem Körperteil des Trägers angeordnet wird.

### Bezugszeichenliste

- 2: Gurt
- 4: textiler Anteil
- 6: Kunststoffelement
- 8: erstes Formschlusselement
- 10: zweites Formschlusselement
- 12: Befestigungselement
- 14: Umlenkelement
- 16: Hinterschneidung
- 18: Bereich

## Patentansprüche

1. Verfahren zum Festlegen einer orthopädietechnischen Einrichtung an einem Körperteil eines Trägers, wobei die Einrichtung
- einen Grundkörper, von dem wenigstens ein Umlenkelement (14) mit einer Anlagefläche in einer Richtung vom Körperteil des Trägers wegweisend hervorsteht,
- wenigstens einen Gurt (2) und
- wenigstens ein Befestigungselement (12) aufweist,
wobei das Verfahren folgende Schritte aufweist:
a) Positionieren der orthopädietechnischen Einrichtung an dem Körperteil,
b) Anlegen des Gurtes (2) an die Anlagefläche des Umlenkelementes (14), und
c) Befestigen des Gurtes (2) an dem Befestigungselement (12), so dass sich der Gurt (2) entlang zweier Teilstrecken erstreckt,
wobei die erste Teilstrecke von einem festen Ende des Gurtes (2), mit dem er an einem Bauteil oder Element der orthopädietechnischen Einrichtung festgelegt ist, bis zu dem Umlenkelement (14) und
wobei die zweite Teilstrecke von dem Umlenkelement (14) bis zu dem Befestigungselement (12) verläuft,
**dadurch gekennzeichnet, dass**
das Anlegen des Gurtes (3) an die Anlagefläche des Umlenkelementes (14) ausführbar ist, ohne dass dazu ein Ende des Gurtes durch eine Schlaufe oder Öse gefädelt werden muss.

2. Verfahren nach Anspruch 1, **dadurch gekennzeichnet, dass** die Anlagefläche eine Nut oder Hinterschneidung (16) aufweist, in der der Gurt (2) angelegt wird.

3. Verfahren nach Anspruch 1 oder 2, **dadurch gekennzeichnet, dass** der Gurt (2) derart an die Anlagefläche angelegt wird, dass der Gurt das Umlenkelement (14) über wenigstens 25 % bevorzugt wenigstens 40 %, besonders wenigstens 50 % seines Umfangs umgibt.

4. Verfahren nach einem der vorstehenden Ansprüche, **dadurch gekennzeichnet, dass** das Befestigungselement (12) an dem Gurt (2) angeordnet ist.

5. Verfahren nach einem der vorstehenden Ansprüche, **dadurch gekennzeichnet, dass** der Gurt (2) ein erstes Formschlusselement (8) aufweist und das Befestigungselement (12) ein zweites Formschlusselement (10) aufweist, die beim Befestigen des Gurtes (2) derart miteinander in Eingriff gebracht werden, dass eine formschlüssige Verbindung entsteht.

6. Orthopädietechnische Einrichtung zum Durchführen eines Verfahrens nach einem der vorstehenden Ansprüche mit einem Grundkörper, von dem wenigstens ein Umlenkelement (14) mit einer Anlagefläche hervorsteht, wenigstens einem Gurt (2) und wenigstens einem Befestigungselement (12), wobei der Gurt (2) angeordnet und eingerichtet ist, an die Anlagefläche des Umlenkelementes (14) angelegt und an dem Befestigungselement (12) befestigt zu werden, ohne dass dazu ein Ende des Gurtes durch eine Schlaufe oder Öse gefädelt werden muss.

7. Orthopädietechnische Einrichtung nach Anspruch 6, **dadurch gekennzeichnet, dass** der wenigstens eine Gurt (2) eine Breite hat, die in wenigstens einem Bereich, der mit der Anlagefläche in Kontakt kommen kann, reduziert ist.

8. Orthopädietechnische Einrichtung nach Anspruch 7, **dadurch gekennzeichnet, dass** die Breite in dem Bereich (18) einseitig reduziert ist.

9. Orthopädietechnische Einrichtung nach Anspruch 7 oder 8, **dadurch gekennzeichnet, dass** der Bereich (18) mit reduzierter Breite länger, bevorzugt wenigstens 1 cm länger, besonders bevorzugt wenigstens 2 cm länger ist, als ein Kontaktbereich des Gurtes (2), mit dem der Gurt (2) mit der Anlagefläche in Kontakt kommt.

10. Orthopädietechnische Einrichtung nach einem der Ansprüche 7 bis 9, **dadurch gekennzeichnet, dass** der Bereich (18) durch ein Kunststoffelement (6), insbesondere ein Spritzgussteil, gebildet wird.

11. Orthopädietechnische Einrichtung nach einem der Ansprüche 10, **dadurch gekennzeichnet, dass** der Gurt (2) einen textilen Abschnitt (4) aufweist, an dem das Kunststoffelement (6) angeordnet ist, wobei bevorzugt das Kunststoffelement (6) ein freies Ende des Gurtes (2) bildet.

12. Orthopädietechnische Einrichtung nach einem der Ansprüche 6 bis 9, **dadurch gekennzeichnet, dass** von dem Grundkörper mehrere, bevorzugt mindestens zwei, besonders bevorzugt mindestens drei oder vier Umlenkelemente (14) mit jeweils einer Anlagefläche hervorstehen.

13. Orthopädietechnische Einrichtung nach einem der Ansprüche 6 bis 10, **dadurch gekennzeichnet, dass** wenigstens ein Umlenkelement (14) in Form eines Hakens ausgebildet ist.

## Claims

1. A method for securing an orthopaedic device to a body part of a wearer, the device comprising
- a base body, from which at least one deflection element (14) with a contact surface protrudes in a direction away from the body part of the wearer,
- at least one belt (2) and
- at least one securing element (12),
the method featuring the following steps:
a) positioning the orthopaedic device on the body part,
b) placing the belt (2) onto the contact surface of the deflection element (14) and
c) securing the belt (2) on the securing element (12) so that the belt (2) extends along two sections, wherein the first section extends from a fixed end of the belt (2), with which it is secured to a component or element of the orthopaedic device, to the deflection element (14), and wherein the second section extends from the deflection element (14) to the securing element (12), **characterized in that** placing the belt (2) onto the contact surface of the deflection element (14) can be carried out without having to thread one end of the belt through a loop or eyelet.

2. The method according to claim 1, **characterized in that** the contact surface comprises a groove or undercut (16) in which the belt (2) is placed.

3. The method according to claim 1 or 2, **characterized in that** the belt (2) is placed on the contact surface in such a way that the belt surrounds the deflection element (14) over at least 25%, preferably at least 40%, especially at least 50%, of its circumference.

4. The method according to one of the preceding claims, **characterized in that** the securing element (12) is arranged on the belt (2).

5. The method according to one of the preceding claims, **characterized in that** the belt (2) comprises a first form-fitting element (8) and the securing element (12) a second form-fitting element (10) that are engaged when fastening the belt (2) in such a way that a form-fitting connection is created.

6. An orthopaedic device for carrying out a method according to one of the preceding claims with a base body, from which at least one deflection element (14) with a contact surface protrudes, at least one belt (2) and at least one securing element (12), the belt (2) being arranged and configured to be placed onto the contact surface of the deflection element (14) and fastened to the securing element (12), without having to thread one end of the belt through a loop or eyelet.

7. The orthopaedic device according to claim 6, **characterized in that** the at least one belt (2) has a width that is reduced in at least one area which can come into contact with the contact surface.

8. The orthopaedic device according to claim 7, **characterized in that** the width in the area (18) is reduced on one side.

9. The orthopaedic device according to claim 7 or 8, **characterized in that** the area (18) with reduced width is longer, preferably at least 1cm longer, especially preferably at least 2cm longer, than a contact area of the belt (2) via which the belt (2) comes into contact with the contact surface.

10. The orthopaedic device according to one of the claims 6 to 9, **characterized in that** the area (18) is formed by a plastic element (6), especially an injectionmolded part.

11. The orthopaedic device according to claim 10, **characterized in that** the belt (2) features a textile section (4) on which the plastic element (6) is arranged, the plastic element (6) preferably forming a free end of the belt (2).

12. The orthopaedic device according to one of the claims 6 to 9, **characterized in that** multiple, preferably at least two, especially preferably at least three or four, deflection elements (14), each with a contact surface, protrude from the base body.

13. The orthopaedic device according to one of the claims 6 to 10, **characterized in that** at least one deflection element (14) is designed in the form of a hook.

## Revendications

1. Procédé de fixation d'un dispositif orthopédique sur une partie du corps d'un utilisateur, le dispositif comportant
- un corps de base d'où fait saillie au moins un élément de renvoi (14), muni d'une surface d'appui, en s'étendant dans une direction s'éloignant de la partie du corps de l'utilisateur,
- au moins une sangle (2), et
- au moins un élément de fixation (12),
le procédé comprenant les étapes suivantes consistant à :
a) positionner le dispositif orthopédique sur la partie du corps,
b) appliquer la sangle (2) contre la surface d'appui de l'élément de renvoi (14), et
c) fixer la sangle (2) à l'élément de fixation (12), de telle sorte que la sangle (2) s'étende le long de deux tronçons partiels,
le premier tronçon partiel s'étendant depuis une extrémité fixe de la sangle (2), par laquelle elle est fixée à un composant ou à un élément du dispositif orthopédique, jusqu'à l'élément de renvoi (14), et
le deuxième tronçon partiel s'étendant depuis l'élément de renvoi (14) jusqu'à l'élément de fixation (12),
**caractérisé en ce que** l'application de la sangle (3) contre la surface d'appui de l'élément de renvoi (14) peut être effectuée sans qu'il soit nécessaire, pour cela, d'enfiler une extrémité de la sangle à travers une boucle ou un œillet.

2. Procédé selon la revendication 1,
**caractérisé en ce que** la surface d'appui présente une rainure ou une contredépouille (16) dans laquelle la sangle (2) est appliquée.

3. Procédé selon la revendication 1 ou 2,
**caractérisé en ce que** la sangle (2) est appliquée contre la surface d'appui de telle sorte que la sangle entoure l'élément de renvoi (14) sur au moins 25 %, de préférence sur au moins 40 %, et en particulier sur au moins 50 % de sa circonférence.

4. Procédé selon l'une des revendications précédentes,
**caractérisé en ce que** l'élément de fixation (12) est disposé sur la sangle (2).

5. Procédé selon l'une des revendications précédentes,
**caractérisé en ce que** la sangle (2) comporte un premier élément de liaison par complémentarité de forme (8), et l'élément de fixation (12) comporte un deuxième élément de liaison par complémentarité de forme (10), qui sont mis en prise l'un avec l'autre lors de la fixation de la sangle (2) de telle sorte qu'il en résulte une liaison par complémentarité de forme.

6. Dispositif orthopédique destiné à mettre en œuvre un procédé selon l'une des revendications précédentes,
comprenant un corps de base d'où fait saillie au moins un élément de renvoi (14) muni d'une surface d'appui, au moins une sangle (2) et au moins un élément de fixation (12), la sangle (2) étant agencée et conçue pour être appliquée contre la surface d'appui de l'élément de renvoi (14) et pour être fixée à l'élément de fixation (12) sans qu'il soit nécessaire, pour cela, d'enfiler une extrémité de la sangle à travers une boucle ou un œillet.

7. Dispositif orthopédique selon la revendication 6,
**caractérisé en ce que** ladite au moins une sangle (2) présente une largeur qui est réduite dans au moins une zone susceptible d'entrer en contact avec la surface d'appui.

8. Dispositif orthopédique selon la revendication 7,
**caractérisé en ce que** la largeur est réduite d'un côté dans ladite zone (18).

9. Dispositif orthopédique selon la revendication 7 ou 8,
**caractérisé en ce que** la zone (18) de largeur réduite est plus longue, de préférence d'au moins 1 cm, et de manière particulièrement préférée d'au moins 2 cm, qu'une zone de contact de la sangle (2) par laquelle la sangle (2) entre en contact avec la surface d'appui.

10. Dispositif orthopédique selon l'une des revendications 7 à 9, **caractérisé en ce que** ladite zone (18) est formée par un élément en matière plastique, en particulier par une pièce moulée par injection.

11. Dispositif orthopédique selon l'une des revendications 6 à 10, **caractérisé en ce que** la sangle (2) comporte une partie textile (4) sur laquelle est disposé l'élément en matière plastique (6), l'élément en matière plastique (6) constituant de préférence une extrémité libre de la sangle (2).

12. Dispositif orthopédique selon l'une des revendications 6 à 9, **caractérisé en ce que** plusieurs éléments de renvoi (14), de préférence au moins deux, de manière particulièrement préférée au moins trois ou quatre, munis chacun d'une surface d'appui, font saillie à partir du corps de base.

13. Dispositif orthopédique selon l'une des revendications 6 à 10, **caractérisé en ce qu'**au moins un élément de renvoi (14) est réalisé sous la forme d'un crochet.
